# EUROPEAN PATENT APPLICATION

(11) **EP 4 029 436 A1**
(43) Date of publication of application: **20.07.2022**
(21) Application number: 21382029.3
(22) Date of filing: 18.01.2021
(51) Int. Cl.: A61B 5/022, A61B 5/021, A63B 21/00, A61B 17/132

(54) **A BLOOD FLOW RESTRICTION SYSTEM, A METHOD FOR PERFORMING BLOOD FLOW RESTRICTION EXERCISE, AND A COMPUTER PROGRAM**

(71) Applicant: Fundació Institut de Ciències Fotòniques, 08860 Castelldefels (ES); Institució Catalana De Recerca I Estudis Avançats (ICREA), 08010 Barcelona (ES)
(72) Inventor: DURDURAN, Turgut, 08860 Castelldefels (ES); KARADENIZ, Umut, 08860 Castelldefels (ES); VERMA, Manish, 08860 Castelldefels (ES); DRAGOJEVIC, Tanja, 08860 Castelldefels (ES); KOBAYASHI FRISK, Lisa, 08860 Castelldefels (ES)
(74) Representative: Ponti & Partners, S.L.P

(57) **Abstract**

The present invention relates to a blood flow restriction system, comprising:
- a tourniquet cuff (C) or band to be placed around a limb, proximally to a target muscle, tightened so as to apply a specific pressure during a blood flow restriction exercise regimen; and
- measuring and monitoring means (M, P) made and arranged to measure and monitor microvascular blood flow within the target muscle, to guide safety and performance parameters of the blood flow restriction exercise regimen according to hemodynamic criteria.

The present invention also relates to a method adapted to use the system of the invention, and to a computer program with code instructions to implement the steps of the method of the invention.

## Description

### FIELD OF THE INVENTION

The present invention generally relates, in a first aspect, to a blood flow restriction system, for performing a blood flow restriction exercise regimen, and particularly to a system configured to guide the exercise regimen from direct microvascular blood flow measurements performed, preferably non-invasively, in the target muscle.

A second aspect of the present invention relates to a method adapted to use the system of the first aspect.

A third aspect of the present invention relates to a computer program with code instructions to implement the steps of the method of the second aspect.

### BACKGROUND OF THE INVENTION

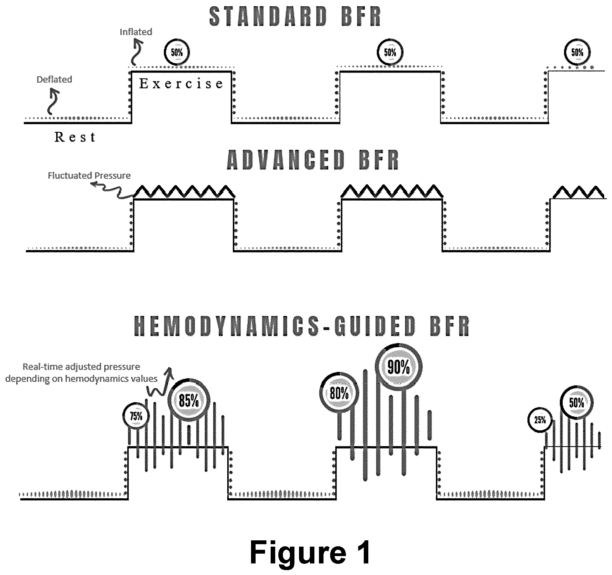

The current state of the art regarding blood flow restriction (BFR) systems is illustrated in Fig 1, top and intermediate views. Top row describes a "standard BFR" regimen where an exercise duration/type is selected and a cuff pressure is pre-determined. This is often done as a percent of the arterial occlusion pressure, i.e. the cuff pressure at which arterial inflow to the target muscles is completely ceased. More advanced methods (Fig 1, middle row) use a similar approach but they modulate the cuff pressure with proprietary algorithms that appear to be pre-determined and cyclic around a pre-determined mean value [US8021283B2].

Others provide estimation of the limb occlusion pressure from a measured physiological parameter.

That is the case of Lima-Soares et al., "Determining the Arterial Occlusion Pressure for Blood Flow Restriction: Pulse Oximeter as a New Method Compared With a Handheld Doppler", 2020, which discloses a blood flow restriction system for performing a blood flow restriction exercise regimen with the features of the preamble of claim 1, i.e. a system comprising:
- a tourniquet cuff or band configured and arranged to be placed around a limb, proximally to a target muscle to be exercised, tightened so as to apply a specific pressure to said limb to restrict arterial blood flow into the limb during a blood flow restriction exercise regimen; and
- measuring and monitoring means to measure and monitor a physiological variable affected by the restricted arterial blood flow, in order to guide safety and performance parameters of the blood flow restriction exercise regimen.

However, in that system known in the art, the physiological variable measured and monitored is the pulsation of the blood in the arteries due to the cardiac cycle, carried out by a pulse oximeter and used to estimate Limb Occlusion Pressure (LOP), and, in addition to provide many errors and limitations in the pressure estimation process, that system only provides indirect and incomplete measurements of the effects of the pressure applied, not necessarily revealing the true impact of the applied pressure and/or exercise on the subject's blood flow and the health of the blood vessels in the target muscle.

Similar systems, with similar drawbacks, but using, instead of a pulse oximeter, a Doppler ultrasound detector, are also known in the art, such as in Bezerra de Morais et al., "Upper limbs total occlusion pressure assessment: Doppler ultrasound reproducibility and determination of predictive variables". Clin. Physiol. Funct. Imaging 37: 437-441, 2017. These also measure the pulsatile component of the arterial blood.

Having direct and complete measurements of the effects of the pressure applied, and revealing the impact of the applied pressure and/or exercise on the subject's blood flow and the health of the blood vessels in the target muscle is very important, not only in terms of efficiency of the performance of the BFR exercise regimen, but also in terms of safety. In the market, there are many types of occlusion bands for BFR technique users. In many cases, depending on the width of the cuff, the performance effect of BFR and safety conditions can change. The safety issue arises from three damaging events: (1) over-compression on the limbs may be harmful for vessels leading to hemorrhages, furthermore, high pressure may induce arterial occlusion, leading to formation of thrombosis in microvasculature, even after reperfusion; (2) compression combined with long periods of BFR and exercise may cause hypoxic-ischemic injury possibly leading to permanent damage; and, (3) repeated tightening/loosening cycles may lead to the dislodging of small blockages (e.g. plaques) which could then cause embolic ischemia in other parts of the body such as the heart and the brain.

It is therefore necessary to provide an alternative to the state of the art which covers the gaps found therein, by providing a BFR system which indeed provides measurements revealing the impact on the subject's blood flow and vessel's health of the BFR exercise regimen, in the target muscle, to guide safety and performance parameters of the blood flow restriction exercise regimen in a personalized manner.

### SUMMARY OF THE INVENTION

To that end, the present invention relates, in a first aspect, to a blood flow restriction system, for performing a blood flow restriction exercise regimen (such as a therapy, a rehabilitative, a training exercise regimen, a targeted development of muscle, or a performance improvement), comprising:
- a tourniquet cuff or band (or other means of restricting the arterial blood flow to the tissue, such as a cord) configured and arranged to be placed around a limb, proximally to a target muscle to be exercised, tightened so as to apply a specific pressure (limb restriction/occlusion pressure) to said limb to restrict arterial blood flow into the limb during a blood flow restriction exercise regimen; and
- measuring and monitoring means to measure and monitor a physiological variable affected by the restricted arterial blood flow, in order to guide safety and performance parameters of the blood flow restriction exercise regimen.

In contrast to the systems known in the prior art, in the system of the first aspect of the present invention, in a characterizing manner, the measuring and monitoring means are made and arranged to measure and monitor microvascular blood flow within at least the target muscle, in order to guide said safety and performance parameters of the blood flow restriction exercise regimen according at least to hemodynamic criteria.

Hemodynamic criteria can be based on microvascular blood oxygenation, volume, flow, oxygen extraction fraction, metabolic rate of oxygen extraction at different time points, in response to occlusion or its release, or their temporal evolution. Both static and dynamic components including variations, e.g. pulsatile, due to the cardiac cycle can be considered. All these variables are, preferably, measured non-invasively and at the target muscle at depths greater than one millimetre and less than three centimetres from the skin surface.

Generally, the measuring and monitoring means are made and arranged to measure and monitor microvascular blood flow within a target muscle that is placed on the same limb whose arterial blood flow is being restricted, but distal to the tourniquet cuff or band.

According to the present invention, by combining one or more BFR exercise regimens with a quantitative measure of target biomarkers, particularly microvascular blood flow within at least the target muscle, measured with the system of the first aspect of the present invention, the three damaging events identified in the above section can be eliminated or at least highly reduced. This is particularly pertinent to when using BFR in injured individuals and in the elderly.

For an embodiment, the tourniquet band (cord, or other equivalent element) is adjustable in diameter, automatically or by a user, so that the diameter of the band can be adjusted to apply more or less pressure to the limb (for the arterial blood flow restriction), guided by the monitored measurements of microvascular blood flow within at least the target muscle.

According to an embodiment, the system further comprises:
- pressure means operatively connected to said tourniquet cuff to at least tightening the same (for example, by inflating the same if it is inflatable) to said specific pressure to restrict arterial blood flow into the limb during said blood flow restriction exercise regimen; and
- a control unit configured and arranged to control one or more parameters (from the above mentioned safety and performance parameters, or derivations thereof) of the blood flow restriction exercise regimen, wherein the control unit is operatively connected to the pressure means to control the same to at least make them tighten and/or loosen the tourniquet cuff (for example, by inflating and/or deflating the same if it is inflatable) according to said one or more parameters of the blood flow restriction exercise regimen.

Those parameters of the blood flow restriction exercise regimen include one or more of the parameters of the following non-exhaustive list of parameters: high pressure (said specific pressure and/or different specific pressures), low pressure, duration time for the application of high pressure (arterial blood flow restriction), duration time for the application of low pressure (without fully restricting the arterial blood flow, or arterial blood flow restriction but with a lower pressure than with the limb occlusion pressure), number of repetition/cycles of the exercise regimen, rest times, intensity and/or type of the exercise.

For an implementation of that embodiment, the measuring and monitoring means are made and arranged to measure and monitor microvascular blood flow within at least the target muscle, at least during part of the blood flow restriction exercise regimen, continuously or at discrete intervals, preferably in real time or near real time.

For another implementation of that embodiment, alternative or complementary to the above described implementation, the measuring and monitoring means are made and arranged to measure and monitor microvascular blood flow within at least the target muscle, at least prior and after the blood flow restriction exercise regimen, so that the effect of the exercise regimen can be determined by comparing the prior and after monitored measurements.

According to an embodiment, the measuring and monitoring means are operatively connected to the control unit, and the control unit implements a closed-loop arrangement using as feedback the measured values received from the measuring and monitoring means to determine one or more values for said one or more parameters of the blood flow restriction exercise regimen, according at least to the hemodynamic criteria, and to automatically control the pressure means to tighten and/or loosen the tourniquet cuff according to the determined value(s) of the one or more parameters of the blood flow restriction exercise regimen.

For an alternative embodiment, for implementing an open-loop arrangement, or for an embodiment complementary to the above described closed-loop arrangement embodiment, the measuring and monitoring means are configured to:
- provide the measured values to a user interface (such as a display, a speaker, a light source, etc.) so that the user can determine one or more values for said one or more parameters of the blood flow restriction exercise regimen, according at least to the hemodynamic criteria, or
- to determine the one or more values for the one or more parameters of the blood flow restriction exercise regimen (by processing the measured values, according at least to the hemodynamic criteria) and provide the determined one or more values to the user interface,
and the control unit comprises input means (such as a keyboard, a mouse, a touchscreen, etc.) for the user to provide the control unit with determined value(s) of the one or more parameters, to make the control unit to control the pressure means to tighten and/or loosen the tourniquet cuff according to the determined value(s) of the one or more parameters of the blood flow restriction exercise regimen.

For a variation of said embodiment, the user interface is an electronic device, such as a smart speaker, which acts as an artificial coach/therapist for the user, guiding him/her in the BFR exercise regimen, with voice instructions (or other kind of signals).

Also, for another embodiment, instead or in addition to the user interface, a further electronic device is operatively connected to the measuring and driving means (or to the control unit) to be controlled based on the received measured values or determined parameter values. Such an electronic device could be, for example, a static bicycle (or another training machine) automatically increasing/reducing the pedal resistance based on the received values.

According to a preferred embodiment, the measuring and monitoring means use a non-invasive technique to quantitatively measure and monitor the microvascular blood flow within at least the target muscle.

For an implementation of that preferred embodiment, the non-invasive technique is a non-invasive optical technique.

The non-invasive optical technique includes, for a preferred variant of said implementation, at least one light (generally, laser) speckle based hemodynamics measurement technique, such as diffuse correlation spectroscopy, near-infrared spectroscopy (NIRS), speckle contrast optical spectroscopy (SCOS), and/or derivatives of those techniques. The measurements reflect the status of the hemodynamics of the microvasculature (including, but not limited to, arterioles, capillaries and venules) at a depth of millimetres to several centimetres, particularly above one millimetre and below three centimetres.

According to an embodiment, the measuring and monitoring means are also made and arranged to measure and monitor one or more of the following further physiological variables, generally from the microvasculature (or microcirculation): blood oxygen saturation, blood volume, blood flow, oxygen extraction fraction and metabolic rate of oxygen consumption, in order to guide the safety and performance parameters of the blood flow restriction exercise regimen according to the above mentioned hemodynamic criteria and also according to a further criteria related to said further physiological variable(s). Further criteria could include other systemic physiological variables such as the resting heart rate as well as its changes that may or may not be deduced from the optical measurements, respiration rate and others. These criteria can be linked to the hemodynamic criteria obtained directly from the muscles.

Different implementations of that embodiment can be carried out, consisting in variations of the above described open-loop and closed-loop arrangement embodiments, to provide (to the user interface or to the control unit) the measured and monitored values of the further physiological variable(s), determine (the measuring and monitoring means, the user interface, or the control unit), based also on those values, the value(s) of the one or more parameters of the blood flow restriction exercise regimen using also the further criteria, and control (the control unit) the pressure means to tighten and/or loosen the tourniquet cuff according to the determined value(s).

For an embodiment, the control unit is configured to control the pressure means to tighten and/or loosen the tourniquet cuff according to a plurality of determined values for the one or more parameters, from measured values provided by the measuring and monitoring means, following a modulation process for a user in a personalized manner.

In this manner, the BFR exercise regimen is adapted to each user, as the effects of the restriction actions (and of the whole BFR exercise regimen) on the target muscle are measured and monitored, prior and after the exercise regimen, or, preferably, during part or the entire exercise regimen.

According to an embodiment, the measuring and monitoring means comprises at least one probe communicating with a wearable or portable device, adapted to be removably attached at least to the target muscle with a position and orientation that allows the same to take the measurements of microvascular blood flow within the target muscle.

For an implementation of that embodiment, the at least one probe is integrated into the tourniquet cuff, while for another implementation (alternative or complementary to that implementation) the at least one probe is attached to a wearable or portable device that is separate, and generally distal, from the tourniquet cuff.

Different computing entities (computers, tablets, smartphones, etc.) can be used, according to different embodiments, for implementing the processing capabilities of the measuring and monitoring means and of the control unit, whether including local and/or remote computing entities, communicated through corresponding communication links (wired or wirelessly), for distributed or non-distributed computing.

For an embodiment of the system of the first aspect of the present invention, the measuring and monitoring means are also configured and arranged to measure and monitor a physiological variable of at least one non-restricted muscle that is not affected by the restricted arterial blood flow but affected by the exercise, also in order to guide safety and performance parameters of the blood flow restriction exercise regimen.

The present invention also relates, in a second aspect, to a method for performing a blood flow restriction exercise regimen, comprising the following steps:
- placing a tourniquet cuff or band (or other means of restricting the arterial blood flow to the tissue, such as a cord) around a limb, proximally to a target muscle to be exercised, tightened so as to apply a specific pressure to said limb to restrict arterial blood flow into the limb during a blood flow restriction exercise regimen; and
- measuring and monitoring a physiological variable affected by the restricted arterial blood flow, in order to guide safety and performance parameters of the blood flow restriction exercise regimen.

In contrast to the methods of the prior art, in the method of the second aspect of the present invention the step of measuring and monitoring a physiological variable comprises measuring and monitoring microvascular blood flow within at least the target muscle, preferably non-invasively, in order to guide said safety and performance parameters of the blood flow restriction exercise regimen according at least to hemodynamic criteria.

The method of the second aspect of the present invention comprises implementing the method steps with the system of the first aspect, for different embodiments.

In a third aspect, the present invention also relates to a computer program, including code instructions that when executed on processors of at least the measuring and monitoring means of the system of the first aspect, implement the steps of the method of the second aspect, to at least measure and monitor a physiological variable affected by the restricted arterial blood flow.

For an embodiment of the computer program of the third aspect of the present invention, the computer program includes further code instructions that when executed on at least one processor of the control unit of the system of the first aspect implement the steps of the method of the second aspect, including those steps carried out by the control unit to at least control the one or more parameters of the blood flow restriction exercise regimen, and to control the pressure means to tighten and/or loosen the tourniquet cuff according to the one or more parameters of the blood flow restriction exercise regimen, and, for the closed-loop arrangement, also those steps carried out by the control unit to determine the one or more values for the one or more parameters of the blood flow restriction exercise regimen, according at least to the hemodynamic criteria (and optionally also according to the further criteria).

The present invention provides several advantages, some or all of the ones indicated below, depending on the embodiment or implementation of the invention:
- Improve blood flow restriction (BFR) regimens with a smart system.
- Guide BFR exercise regimen based on the muscle microvasculature function of the target muscles for training.
- Muscle hemodynamics and oxygen metabolism are measured by using safe levels of light.
- Non-invasive, continuous (or pseudo-continuous), rapid (up to tens of Hertz) measurements.
- Muscle probe(s) suitable for use on the arm, palm, leg, foot muscles distal to the occlusion point and, optionally, additional ones suitable for use on other accessible muscles such as the back or neck muscles.
- Communication with BFR band with ability to control restriction, tightening timing and pressure.
- Real-time analysis and interpretation modules in software and/or hardware/firmware.
- Wired or wireless data transfer to mobile devices, watches, cloud computing.
- Skin and eye-safe.
- Easy-to-use.
- Cost-effective.
- Personalized BFR exercise regimens that learn as one progresses over time as well as during a specific session.
- Muscle exercise specific regimens.
- Enhanced safety for minimizing hypoxic-ischemic injury, haemorrhages, blood coagulation and muscle damage.

### BRIEF DESCRIPTION OF THE FIGURES

In the following some preferred embodiments of the invention will be described with reference to the enclosed figures. They are provided only for illustration purposes without however limiting the scope of the invention.
Figure 1 schematically shows, for comparison some diagrams indicating pressure changes in BFR mechanisms of the prior art (top and intermediate views) and for the present invention (bottom view), for an embodiment, during exercising and resting period of an exercise regimen.
Figure 2 shows diagrams for Left and Right forearms, displaying different hemodynamic responses, specifically Blood Flow (BF) and absolute tissue oxygen saturation (StO2), in response to equal pressure occlusion on the same subject.
Figure 3 depicts a diagram showing different blood flow responses to equal applied occlusion pressure for two different subjects (Sub land Sub2) obtained with a commercially available BFR device.
Figure 4 depicts two diagrams which show how applying incremental increases in occlusion pressure on the arms of different subjects shows varying inter-subject responses in both blood flow and tissue oxygen saturation as measured by diffuse optics.
Figure 5 depicts several diagrams showing pulsatile signals with the same cuff occlusion pressure (0, 50, 80 and 110 mmHg) for subject 1 (top panel/row) and subject 2 (bottom panel/row). In these panels, the pulsatile signal until the third harmonics is visible at lower occlusion pressures. However, in the top panel at 110 mmHg, there is no detectable pulsatile signal, showing importance for monitoring the pulsatile signal during cuff occlusion.
Figure 6 schematically shows the system of the first aspect of the present invention, for an embodiment for implementing an open-loop arrangement.
Figure 7 schematically shows the system of the first aspect of the present invention, for an embodiment for implementing a closed -loop arrangement.
Figure 8 schematically shows the system of the first aspect of the present invention, for a variation of the embodiment shown in Figure 7.
Figure 9 schematically shows the system of the first aspect of the present invention, for a slight variation of the embodiment shown in Figure 8.
Figure 10 shows a flowchart for implementing different steps of the method of the second aspect of the present invention or actions of the system of the first aspect, for an embodiment.
Figure 11 shows a flowchart very similar to that of Figure 10, but with a slight variation, to specify that the limb occlusion pressure is determined for a pulsatile signal, for another embodiment.
Fig. 12 schematically shows an example for probe placement on the arm muscle, according to an embodiment of the present invention.
Fig. 13 schematically shows another example for probe placement on the two different leg muscles, for another embodiment of the present invention.

### DESCRIPTION OF THE PREFERRED EMBODIMENTS

In the present section some embodiments of the present invention will be described with reference to the appended Figures.

As already textually described in a previous section, but graphically disclosed in Figure 1, the present invention (bottom view) differs from those of the prior art (top and intermediate views) in that the target muscle hemodynamics, during and/or prior and after the BFR exercise regimen, optionally enhanced by other physiological information from wellness monitors (e.g., heart-rate, respiration-rate, etc.) and non-BFR exercise monitors, will be used to determine the cuff pressure, preferably in real-time, based on target hemodynamic values. This is illustrated in Fig 1, bottom view. This combination of features indeed provides enhanced efficacy for BFR exercise regimens, specifically hemodynamics-guided BFR exercise regimens.

The present invention provides a semi-automated (open-loop arrangement) or fully automated (closed-loop arrangement) and "smart" solution for personalized, goal-oriented BFR exercise regimens which compares well against methods that are manual and take a "one-size-fits-all" approach, and also against those that offer minimal personalization such as those with a built-in measure of the limb occlusion pressure to define the cuff amount and those that employ a cyclic modification of the cuff pressure.

Overall, the present invention: (1) simplifies the process, (2) personalizes the BFR exercise regimen, (3) provides higher amount of safety and, (4) increases the efficacy of the BFR exercise regimen.

Preferably, diffuse optical techniques are used to implement the measuring and monitoring means of the system of the first aspect of the present invention, and also of the corresponding steps of the method of the second aspect.

In this sense, diffuse optical techniques have been extensively validated in the past, although not for BFR systems, therefore, it is beyond of the scope of this document to provide evidence to that. The present invention is placed in the context of the hemodynamics guided BFR exercise regimens versus existing methods for BFR exercise regimens.

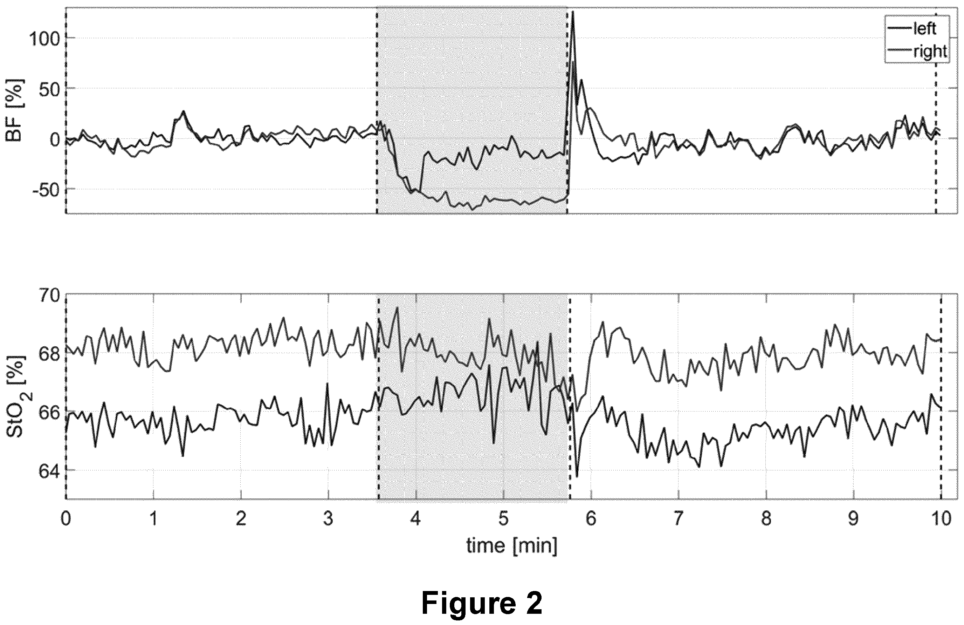

Using diffuse optics, it has been shown, by the present inventors, that simultaneous arm occlusions on the left and right arms with the same occlusion pressure using a commercially available basic BFR band can trigger different hemodynamic responses in the two arms, as shown in Figure 2. This highlights the potential relevance of a technique that can provide real-time feedback on the efficacy of BFR occlusion.

Specifically, in Figure 2, the top row shows a time trace of changes in relative blood flow (BF), relative to baseline, measured using DCS (Diffuse Correlation Spectroscopy). The shaded area represents the time at which the occlusion pressure was applied. The bottom row shows a time trace of changes in absolute tissue oxygen saturation (StO2) using NIRS. In each time trace, the responses in left and right forearm after an equal occluding pressure was applied on left and right upper arms are plotted. It is seen that there was a greater decrease in the blood flow in the right forearm (-70%) compared to the left forearm (-20%). The tissue oxygenation saturation showed a gradual decrease in the right arm during cuff occlusion (-2% decrease) while no such decrease was observed on the left arm. Together the results from the left arm indicate a potential failure of the BFR in restricting blood flow even though the systemic blood pressure was same and BFR was applied according to the manufacturer's instructions. In addition, the tissue oxygen saturation in the right forearm has a slightly higher initial baseline value (-68%) than that in the left forearm (-66%).

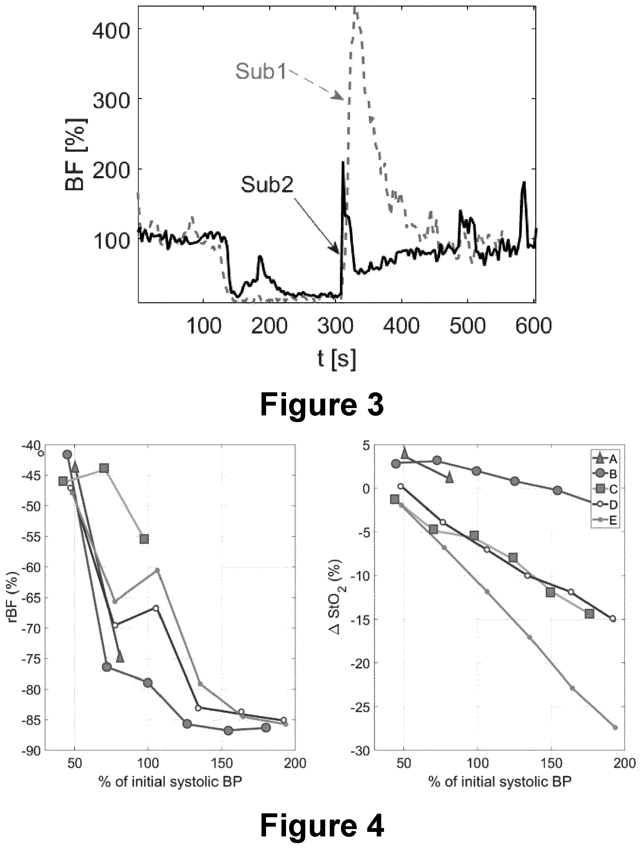

In Figure 3, differences in blood flow response (measured with DCS) to equal occluding pressures in two different subjects (Sub1 and Sub2) are shown in a diagram. As in Figure 2, the blood flow was measured, by the present inventors, in the forearm of each subject and the same commercially available BFR band was placed on the upper arm. The hemodynamic response to the cuff occlusion is noticeably different in the two subjects. In Sub 1, the hyperemic peak of the blood flow reached values exceeding 300% of the baseline value while in Sub 2, the hyperemic peak of the blood flow reached 100% of the baseline value. In addition, in Sub 1, the hyperemic peak occurred with a delay compared to Sub 2.

Figures 2 and 3 demonstrate the intra- and inter-subject variability in hemodynamic responses respectively. This variability is currently not accounted for in existing BFR systems and can potentially address both concerns related to clinical safety as well as guide the BFR regimen to maximize efficacy of exercise/treatment.

Furthermore, in Figure 4 the ability of diffuse optics to detect hemodynamic changes in response to incremental increases (50mmHg, 80mmHg, 110mmHg, 140mmHg, 170mmHg, 200mmHg) of arm cuff occlusion pressure is shown, for different subjects (A, B, C, D, E), from experiments performed by the present inventors. The left plot shows the relative change in blood flow from baseline and the right plot shows the change in tissue oxygenation saturation from baseline. Again, an inter-subject variability of diffuse optical parameters in response to arm cuff occlusion is seen. At the lowest applied occlusion pressures (first data point corresponding to 50 mmHg absolute applied cuff pressure), it is seen that in subjects A and B, the tissue oxygen saturation increased by approximately 3% compared to baseline while the blood flow decreased by approximately 43%. In the other subjects, the tissue oxygen saturation decreased by a similar amount and the relative change in blood flow was slightly higher. At occlusion pressures greater than the systolic blood pressure, the general trends in both blood flow and tissue oxygen saturation become more uniform with all subjects eventually displaying decreases from baseline. However, the rate of decrease in both blood flow and tissue oxygenation saturation is variable. Additionally, the changes in both parameters even at the highest absolute applied occlusion pressure (200mmHg), at which both arterial and venous blood flow is occluded, display a large heterogeneity.

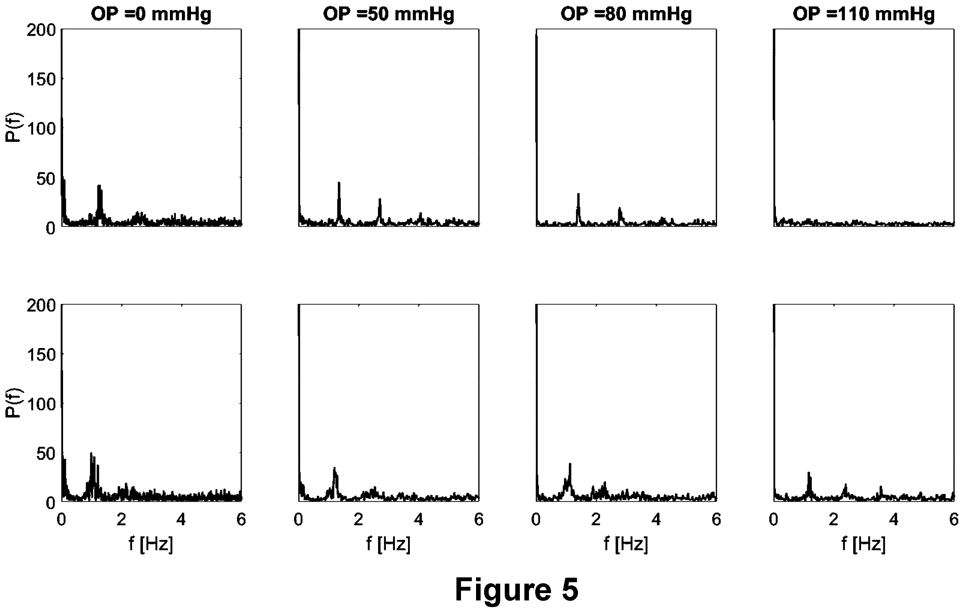

Furthermore, if one looks at the pulsatile blood flow signal, due to the cardiac cycle, in the arm (Figure 5), when there is no occlusion, a distinct pulsatile signal, up to the third harmonics, is clearly visible. When the occlusion pressure (OP) is increased, the peak amplitude of the pulsatile signal decreases successively until it eventually disappears (Figure 5, top panel at 110 mmHg). The occlusion pressure at which the pulsatile signal disappears is related to the systolic blood pressure of the subject, where for subjects with lower systolic pressures (top row, systolic pressure 105mmHg) the pulsatile signal ceases at a lower occlusion pressure (110mmHg) and for subjects with high systolic blood pressures (bottom row, systolic pressure 148mmHg) a higher occlusion pressure is required (greater than 110mmHg). The results from the two subjects in Figure 5, obtained by the present inventors, demonstrate the differences in response with respect to the applied pressure. These results indicate that: (1) the present invention is able to monitor the cessation of arterial (pulsatile) blood flow into the target muscle, hence, guiding the BFR pressure application, (2) there are heterogeneities in this response between subjects which can be monitored by the present invention, and thus personalize the BFR exercise regimen to each subject.

The calibration pressure (also known as "individualized pressure calibration") in these kinds of systems is usually standard for both limbs. According to the results obtained by the present inventors, different amounts of pressure are necessary to get the optimized results for different limbs. Therefore, the present invention does not only provide a personalization of the BFR exercise regimen for each subject, but also for each limb of the same subject.

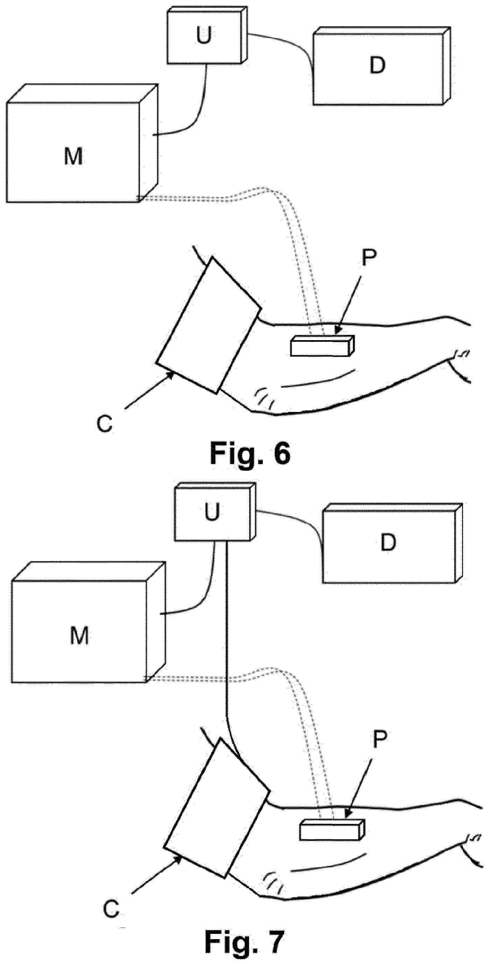

Different implementations of the system of the first aspect of the present invention are shown in Figures 6 to 9, all of them having in common that relate to a BFR system, for performing a blood flow restriction exercise regimen, comprising:
- a tourniquet cuff C or band configured and arranged to be placed around a limb, proximally to a target muscle to be exercised, tightened so as to apply a specific pressure to said limb to restrict arterial blood flow into the limb during a blood flow restriction exercise regimen; and
- measuring and monitoring means M, P made and arranged to measure and monitor microvascular blood flow within the target muscle, in order to guide safety and performance parameters of the blood flow restriction exercise regimen according at least to hemodynamic criteria.

Also for all the implementations depicted in those Figures 6 to 9, measuring and monitoring means comprise a tourniquet cuff C, a diffuse optical monitor M, with processing/computing capabilities, and the appropriate software installed therein, and, optically and operatively connected thereto, one or more optical probes P appropriately placed on the target muscle, while the control unit U also has processing/computing capabilities, and an appropriate software installed therein, and a display/screen D to present information to the user.

Specifically, Figure 6 shows an embodiment for implementing an open-loop arrangement of the system, such as a stand-alone hybrid (e.g. diffuse optical spectroscopy (DOS) and diffuse correlation spectroscopy (DCS)) or mono-modal (e.g. DOS or DCS only) diffuse optical monitor M, with appropriate tissue probes P that provides hardware and software interfaces with appropriate means (such as an API or SDK). The monitor M will provide instructions for tightening/loosening of the cuff(s) C and for their real-time occlusion adjustments based on its algorithms derived from the measurements of hemodynamics performed with the probe P. At the same time, the real time hemodynamics and pressure values can be monitored on the internal or external system screen D.

Another implementation is shown in Figure 7, for a hybrid or mono-modal diffuse optical monitor M and one or a set of automated cuffs C (only one cuff is shown), in order to provide an all-inclusive BFR system, distinguishing form that of Figure 6 in that this implementation corresponds to a closed-loop arrangement, i.e. that the control unit U is operatively connected to the cuff C (including the pressure means thereof, which are not shown) and automatically controls the parameters of the BFR exercise regimen from the monitored and measured parameter(s). This implementation avoids the use of custom cuff software.

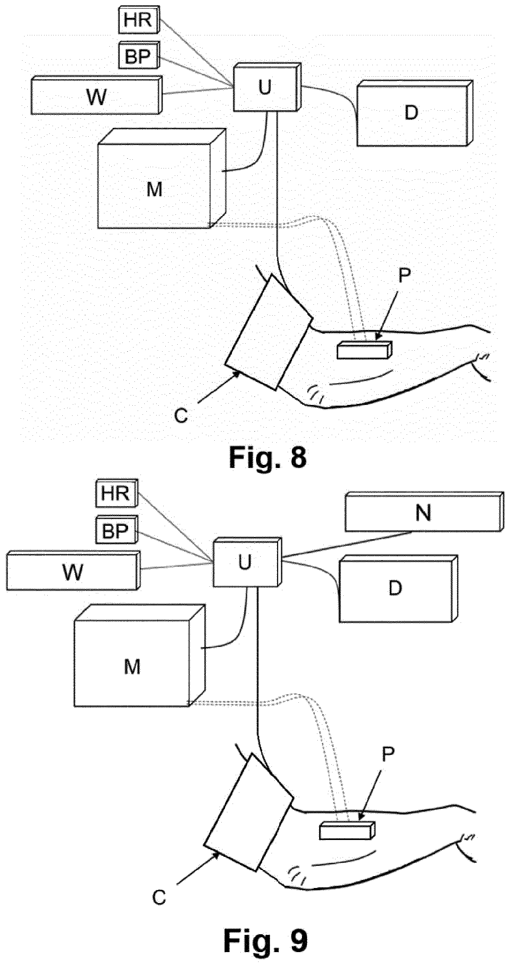

A further implementation is shown in Figure 8, which includes the same components shown in Figure 7, and additional components, such as smart watches W, and other wellness monitoring devices, such as devices for monitoring Blood Pressure (BP) or Heart Rate (HR), operatively connected to the control unit U, to provide the same with values of additional physiological parameters, such as the heart-rate, respiration and arterial oxygen saturation. These values could be used for calculating a reference algorithm or tracking the user performance before/during and/or after BFR exercise regimen.

Figure 9 shows another implementation of the system of the first aspect of the present invention, which includes the same components shown in Figure 8, and also outputs providing information to cloud or mobile infrastructures N about the muscle physiology during the exercise regimen to develop personalized suggestions and to track the progress of the BFR regimen, from remote computing entities connected to those infrastructures N.

All the implementations can be envisioned in:
(a) a simple, low-cost consumer-oriented version with inferior capabilities and quality control,
(b) a safe and more advanced professional use, and,
(c) as a medical device with additional regulatory approvals and specifications to (b).

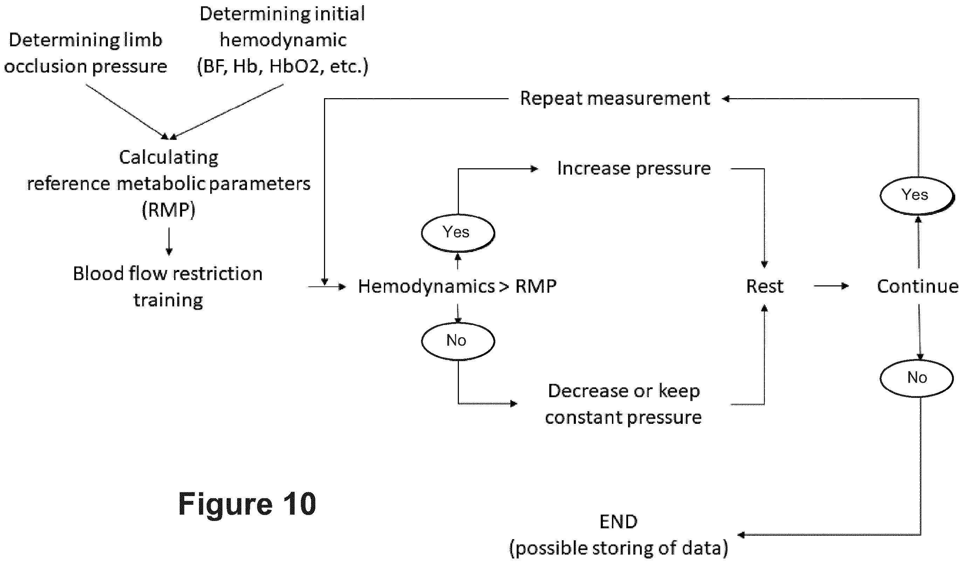

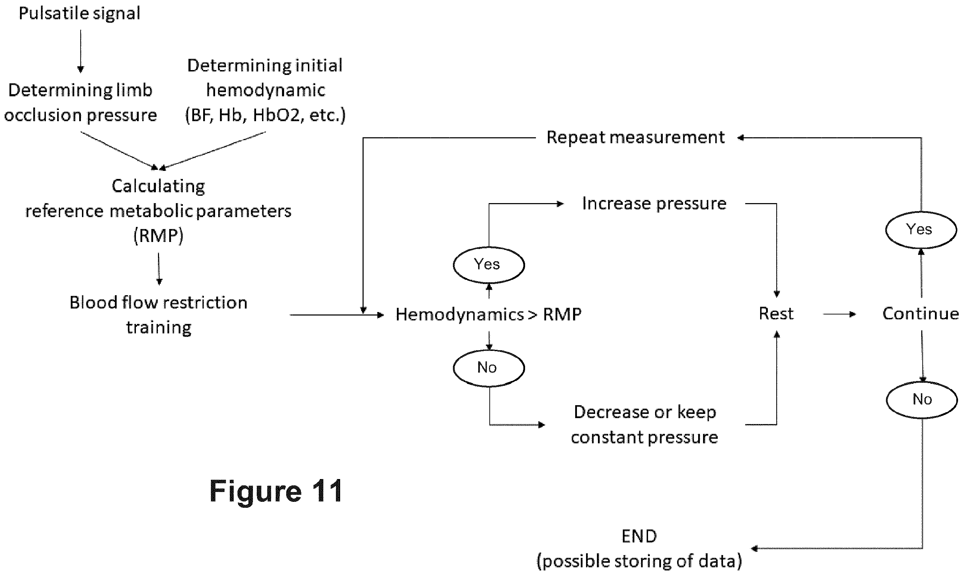

Figures 10 and 11 show two flowcharts for implementing different steps of the method of the second aspect of the present invention or actions of the system of the first aspect, for an embodiment, for which the system/methods uses diffuse optics technology, limb occlusion cuffs, and other physiological monitoring tools to be used for personalized hemodynamics guided-BFR therapy.

According to those flowcharts, of work-flows, hemodynamic parameters including, but not limited to, blood flow (BF) and tissue oxygen saturation are continuously measured in real-time using diffuse optics. Continuous feedback of these signals to the control unit U adjusts in real-time the applied occlusion pressure to the user's personalized ideal value for maximum exercise efficacy as well as serving as a safety monitorization tool. Additional physiological parameters could also be monitored (e.g. heart rate, blood pressure, etc.) simultaneously with general physiological responses to the exercise. All acquired data is collected by a software and synchronized for real-time display to the user/trainer and is also stored for downloading at a later time.

Specifically, in the flowchart of Figure 10 the following steps are carried out:
1. Before the start of the BFR exercise regimen, initial hemodynamic parameters should be calculated (e.g. Hb, HbO2, BF, LOP, etc.).
2. When the initial parameters (hemodynamic parameters and limb occlusion pressure) are set, reference metabolic parameters (RMP) are calculated and BFR exercise can start. Reference metabolic parameters can be calculated as including more parameters or can be evaluated separately.
3. If the measured hemodynamics during BFR regimen is higher than RMP, then the cuff pressure should be increased. Else the pressure should be decreased or kept constant. Then, a rest cycle is performed, and if the user wants to continue with the exercise, at least the hemodynamics measurements are repeated and compared again with the RMP. Otherwise, the regimen is ended.
4. Although not shown in the flowchart, to maximize the BFR efficacy, external parameters such as body mass index (BMI), age, gender, height, blood pressure (systolic and diastolic), muscle circumference, will be added to optimize the reference metabolic parameters in a more personalized manner.

With the real time hemodynamic values, the pressure on the limb can be varied and thus, with provided optimal personalized values, duration and timing of the tightening can be optimized. Portable/wearable hybrid diffuse optical monitoring can provide a personalized BFR regimen and follow-up on the progression of the recovery/exercise during specific sessions and over multiple sessions. The output from the multi-modal monitor could be sent to mobile devices, watches, clouds, allowing real time monitoring. The invention comprising to provide a technique for enabling a person without special knowledge to effectively and safely use a pressurized muscle strength training method.

In figure 11, it is specified that the limb occlusion pressure is determined for a pulsatile signal (a pulsatile blood flow signal), for another embodiment. Particularly, to determine the limb occlusion pressure, a cessation of that pulsatile signal could be used:
- First, the pulsatile signal at 0 mmHg is measured and the peak amplitude is determined.
- Then, the occlusion pressure is increased until the cessation of the pulsatile peak.
- Once there is no more peak, according to a percentage or based on the height of the peak, the LOP is determined.
- Throughout the BFR training, the peak is recalculated making sure that the LOP is correct and adjusted accordingly.
- If blood flow or reference metabolic parameters of the subject increase during the exercising interval, the level of cuff pressure oscillations would increase (on the other hand, if the RMP of the subject goes down while the cuff is tightened, the level of oscillations in the cuff would decrease); in that case, the cuff can be gradually loosened.

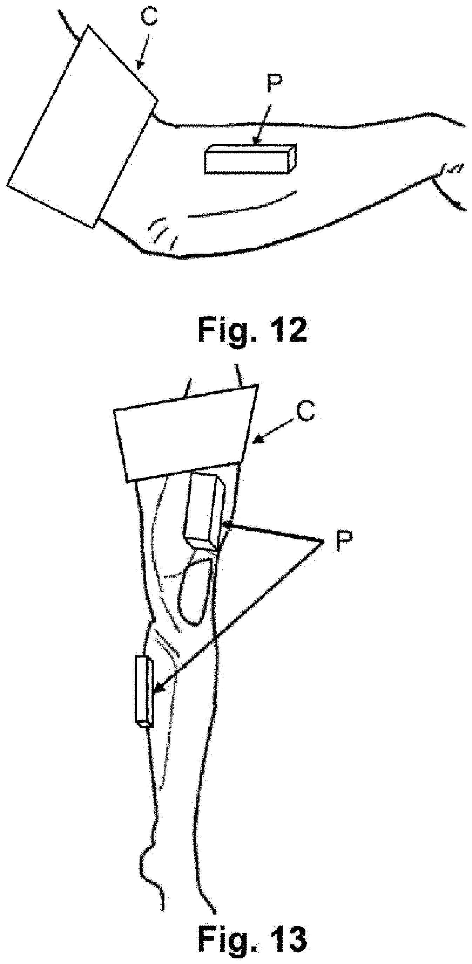

To elaborate the description of the above mentioned "appropriate placement" of the optical probes P on the target muscle, the following explanation is provided for two embodiments, shown in Figures 12 and 13.

The diffuse optical component consists of single or multiple light sources and single or multiple detectors integrated onto a light-weight and skin-friendly probe P that can be placed directly onto the skin of the user at the point of interest e.g. the forearm or leg for non-invasive continuous monitoring of local metabolic parameters (Figures 12 and 13). These parameters aid directly in the adjustment of the limb occlusion cuffs C, and are crucial in personalizing BFR exercise regimens. Current BFR exercise regimens rely on baseline calibration of blood pressure to determine the applied BFR occlusion pressures. However, with real time monitoring of local skeletal muscle metabolism, BFR exercise regimens can be adjusted to stress/nourish the muscle for optimum outcome. The exact outcome is dependent on the user, but can include outcome of physiotherapy after injury or illness or outcome after exercise training.

Measurements can be done anywhere on the body but in general (i.e., on any target muscle being affected by the BFR), the optical probe must be placed on the same target limb, but more distal, to the occluding cuff, such as one probe P for a target muscle of the arm (Figure 12) or two probes P at two different target muscles of the leg affected by the BFR (Figure 13).

In addition to diffuse optical monitoring of metabolic parameters to guide BFR, the other relevant physiological parameters that are recorded and integrated to the system workflow shown in Figures 10 and 11, will allow for non-localized systemic monitoring of the user, such as e.g. blood pressure and heart rate, and other hemodynamic parameters. Integrating systemic monitoring to the BFR device in addition to diffuse optical monitoring will allow for a complete monitoring of the physiology, not only of local metabolism. This is beneficial from the point-of-view of medical safety.

Finally, the system software will allow for real-time acquisition of the data as well as save all data for later download, for some embodiments. The output from the system can be sent to mobile devices such as smart-watches and laptops or upload to cloud.

A person skilled in the art could introduce changes and modifications in the embodiments described without departing from the scope of the invention as it is defined in the attached claims.

## Claims

1. A blood flow restriction system, for performing a blood flow restriction exercise regimen, comprising:
- a tourniquet cuff (C) or band configured and arranged to be placed around a limb, proximally to a target muscle to be exercised, tightened so as to apply a specific pressure to said limb to restrict arterial blood flow into the limb during a blood flow restriction exercise regimen; and
- measuring and monitoring means (M, P) to measure and monitor a physiological variable affected by the restricted arterial blood flow, in order to guide safety and performance parameters of the blood flow restriction exercise regimen;
**characterized in that** said measuring and monitoring means (M, P) are made and arranged to measure and monitor microvascular blood flow within at least said target muscle, in order to guide said safety and performance parameters of the blood flow restriction exercise regimen according at least to hemodynamic criteria.

2. The blood flow restriction system of claim 1, wherein the system further comprises:
- pressure means operatively connected to said tourniquet cuff (C) to at least tightening the same to said specific pressure to restrict arterial blood flow into the limb during said blood flow restriction exercise regimen; and
- a control unit (U) configured and arranged to control at least one parameter of said blood flow restriction exercise regimen, wherein said control unit (U) is operatively connected to said pressure means to control the same to at least make them tighten and/or loosen the tourniquet cuff (C) according to said at least one parameter of the blood flow restriction exercise regimen.

3. The blood flow restriction system of claim 1 or 2, wherein said measuring and monitoring means (M, P) are made and arranged to measure and monitor microvascular blood flow within at least said target muscle, at least during part of the blood flow restriction exercise regimen and/or at least prior and after the blood flow restriction exercise regimen.

4. The blood flow restriction system of claims 2 or 3, when depending on claim 2, wherein said measuring and monitoring means (M, P) are operatively connected to said control unit (U), wherein the control unit (U) implements a closed-loop arrangement using as feedback the measured values received from the measuring and monitoring means (M, P) to determine at least one value for said at least one parameter of the blood flow restriction exercise regimen, according at least to said hemodynamic criteria, and to automatically control the pressure means to tighten and/or loosen the tourniquet cuff (C) according to said at least one determined value of the at least one parameter of the blood flow restriction exercise regimen.

5. The blood flow restriction system of claim 2, 3 or 4, when depending on claim 2, wherein the measuring and monitoring means (M, P) are configured to:
- provide the measured values to a user interface so that the user can determine at least one value for said at least one parameter of the blood flow restriction exercise regimen, according at least to said hemodynamic criteria, or
- determine the at least one value for the said at least one parameter of the blood flow restriction exercise regimen, according at least to the hemodynamic criteria, and provide the determined at least one value to the user interface;
and wherein the control unit (U) comprises input means for said user to provide the control unit (U) with said at least one determined value of the at least one parameter, to make the control unit (U) to control the pressure means to tighten and/or loosen the tourniquet cuff (C) according to said at least one determined value of the at least one parameter of the blood flow restriction exercise regimen.

6. The blood flow restriction system of any of the previous claims, wherein said measuring and monitoring means (M, P) use a non-invasive technique to quantitatively measure and monitor the microvascular blood flow within at least the target muscle.

7. The blood flow restriction system of claim 6, wherein said non-invasive technique is a non-invasive optical technique.

8. The blood flow restriction system of claim 7, wherein said non-invasive optical technique includes at least one light speckle based hemodynamics measurement technique.

9. The blood flow restriction system of any of the previous claims, wherein said measuring and monitoring means (M, P) are also made and arranged to measure and monitor at least one of the following further physiological variables: blood oxygen saturation, blood volume, blood flow, oxygen extraction fraction and metabolic rate of oxygen consumption, in order to guide said safety and performance parameters of the blood flow restriction exercise regimen according to said hemodynamic criteria and also according to a further criteria related to said at least one further physiological variable.

10. The blood flow restriction system of claim 2 or of any of claims 3 to 9, when depending on claim 2, wherein the control unit (U) is configured to control the pressure means to tighten and/or loosen the tourniquet cuff (C) according to a plurality of determined values for said at least one parameter, from measured values provided by the measuring and monitoring means (M, P), following a modulation process for a user in a personalized manner.

11. The blood flow restriction system of any of the previous claims, wherein the measuring and monitoring means (M, P) comprises at least one wearable or portable probe (P) adapted to be removably attached to the target muscle with a position and orientation that allows the same to take said measurements of microvascular blood flow within the target muscle.

12. A method for performing a blood flow restriction exercise regimen, comprising the following steps:
- placing a tourniquet cuff (C) or band around a limb, proximally to a target muscle to be exercised, tightened so as to apply a specific pressure to said limb to restrict arterial blood flow into the limb during a blood flow restriction exercise regimen; and
- measuring and monitoring a physiological variable affected by the restricted arterial blood flow, in order to guide safety and performance parameters of the blood flow restriction exercise regimen;
**characterized in that** said step of measuring and monitoring a physiological variable comprises measuring and monitoring microvascular blood flow within at least said target muscle, in order to guide said safety and performance parameters of the blood flow restriction exercise regimen according at least to hemodynamic criteria.

13. The method of claim 12, **characterized in that** the method comprises implementing said steps with the system according to any of claims 1 to 11.

14. A computer program, including code instructions that when executed on processors of at least the measuring and monitoring means (M, P) of the system, implement the steps of the method of claim 13, to at least measure and monitor a physiological variable affected by the restricted arterial blood flow.

15. The computer program of claim 14, including further code instructions that when executed on at least one processor of the control unit (U) implement the steps of the method according to claim 13 when using the system of claim 2 or of any of claims 3 to 11, when depending on claim 2, including those steps carried out by the control unit (U) to at least control said at least one parameter of the blood flow restriction exercise regimen, and to control the pressure means to tighten and/or loosen the tourniquet cuff (C) according to said at least one parameter of the blood flow restriction exercise regimen.
